# EUROPEAN PATENT SPECIFICATION

(11) **EP 2 997 877 B1**
(45) Date of publication and mention of the grant of the patent: **14.03.2018**
(21) Application number: 15184818.1
(22) Date of filing: 11.09.2015
(51) Int. Cl.: A61B 1/005

(54) **BENDING CONTROL MECHANISM FOR ENDOSCOPE**
BIEGUNGSSTEUERUNGSMECHANISMUS FÜR ENDOSKOP
MÉCANISME DE COMMANDE DE PLIAGE POUR ENDOSCOPE

(30) Priority: 17.09.2014 JP 2014189127
(43) Date of publication of application: 23.03.2016
(73) Proprietor: HOYA Corporation, Tokyo 160-8347 (JP)
(72) Inventor: SAITO, Keiichi, Shinjuku-ku, Tokyo 161-8525 (JP)
(74) Representative: Schaumburg und Partner Patentanwälte mbB

(56) References cited:
- JP-A- 2005 028 018
- JP-A- 2012 081 010
- US-A1- 2008 275 302
- US-A1- 2014 256 489

## Description

### BACKGROUND OF THE INVENTION

### 1. Field of the Invention

The present invention relates to a bending control mechanism of an endoscope.

### 2. Description of the Related Art

In an endoscope in which a bendable section that forms an insertion section can be controllably bent, a bending control mechanism, in which a bending control member is provided on a control body that connects onto a base end of the insertion section and by which the bending angle of the bendable section can be changed by pulling or loosening wires that are connected to a pulley by rotating the pulley by operating the bending control member, is heavily used (used very frequently). The pulling state of the wires is altered by increasing/decreasing the winding amount of the wires on the pulley in accordance with the rotation of the pulley. In such a type of bending control mechanism, since the resistance against a bending operation increases as the amount of bending of the bendable section increases, inventions which reduce the amount of bending-operational force have been proposed in Japanese Unexamined Patent Publication No.H05-329096 (Patent Literature 1) and Japanese Unexamined Patent Publication No.2005-28018 (Patent Literature 2).

Patent Literature 1 discloses an invention that is directed at reducing the load on the bending operation by reducing the diameter of the pulley that forms part of the bending control mechanism. If the diameter of the pulley is reduced, although the amount of bending-operational force for winding the wire onto the pulley can be reduced, due to the wire which is wound onto the pulley being repeatedly extended and bent at a small radius of curvature, problems occur with fatigue occurring in the wire, thereby causing the wire to break. As a countermeasure to such a problem, Patent Literature 1 teaches a configuration in which a winding-attachment section, configured of a plurality of links which are connected to each other in a relatively rotatable manner, is provided that winds onto the pulley, and the drawing-out end of the winding-attachment section (the plurality of links) is attached to the wire, thereby preventing fatigue occurring in the wire.

In Patent Literature 2, a reduction in the amount of bending-operational force is achieved by applying a pulling auxiliary force on the pulley that corresponds to the pulling amount of the wire using a power-assisted motor provided inside the control body.

Both of the inventions disclosed in Patent Literature 1 and 2 contribute to the reduction of the amount of bending-operational force; however, by adding links or a power-assisted motor, the component configuration becomes complex, thereby increasing manufacturing costs and risking a reduction in productivity. Furthermore, as a result of adding such in-built components, the control body becomes larger and heavier, possibly deteriorating the holdability of the control body. Furthermore, in the configuration disclosed in Patent Literature 1, due to the diameter of the pulley being reduced in size, the rotational angle of the pulley increases per unit amount of winding of the wire, and hence, the operational movement amount of the bending-control member becomes greater, thereby increasing the possibility of the person who is operating the endoscope getting a tired hand.

A bending control mechanism for an endoscope according to the preamble of claim 1 is disclosed in JP 2005 028018 A.

US 2008/275302 A1 discloses an endoscope including an insertion portion having a bendable portion and a bending mechanism configured to bend the bendable portion. The bending mechanism includes a pulley, an annular member, a pulling member and a pulling operation member. The annular member is formed into a C-shape with a cut-out and can be elastically deformed. The annular member is externally fitted to the pulley with a gap. The pulling member has an intermediate portion wound around the annular member. The distal end portion of the pulling member is connected to the bending portion, and the proximal end portion of the pulling member is connected to the pulling operation member which pulls the pulling member. The width direction position at the winding start position of the pulling member wound around the annular member is made different from the width direction position at the winding end position of the pulling member.

JP 2012 081010 A discloses a stiffening mechanism including a pulley with eccentric axis of rotation. The outer peripheral profile of the pulley includes a small-radius section and a large-radius section. A wire is wound onto the large-radius section at an initial winding stage and wound onto the small-radius section at a stage where the wire is pulled by a maximum amount.

US 2014/256489 A1 discloses a system for controlling a medical device including a plurality of pulley pieces forming a collapsible pulley.

### SUMMARY OF THE INVENTION

The present invention has been devised in view of the above-mentioned problems and provides a bending control mechanism of an endoscope having a simple structure while achieving a decreased amount of bending-operational force, thereby exhibiting a superior operability.

According to an aspect of the present invention, a bending control mechanism for an endoscope is provided, provided with a control body and a bendable section which includes an insertion section, the bending control mechanism including a pulley which is rotatably provided in the control body; and a wire which is connected between the pulley and the bendable section. An amount of the wire is wound onto the pulley in accordance with a change in rotation of the pulley. An outer peripheral profile of the pulley, onto which the wire is wound, includes a non-circular section including a small-radius section having a small radius from a rotational center axis of the pulley, and a large-radius section having a radius from the rotational center axis that is larger than the small radius. The wire is wound onto the large-radius section at an initial winding stage at which the bendable section is at a non-bending position. The wire is wound onto the small-radius section at a stage where the bendable section is bent by a maximum amount.

It is desirable for the pulley to include a varying-radius section between the small-radius section and the large-radius section, wherein a radius of the varying-radius section from the rotational center axis gradually increases with respect to a direction from the small-radius section to the large-radius section.

It is desirable for the varying-radius section to include a radial connecting section which smoothly connects the small-radius section with the large-radius section without having any stepped profile therebetween. An end of the wire is fixed to the pulley at a position within a range, in a circumferential direction of the pulley, other than the small-radius section, the large-radius section and the radial connecting section.

Each of the small-radius section and the large-radius section of the pulley is a constant radius section having a constant radius from the rotational center axis.

In the bending control mechanism, of an endoscope, according to the present invention, the wire can be wound onto the large-radius section of the pulley when the bending amount of the bending section is small to reduce the rotation operational amount of the pulley per unit amount of bending (so that the wire can be efficiently pulled with a small operational amount) and reduce the bending load on the wire, and the wire can be wound onto the small-radius section of the pulley when the bending amount of the bending section is large to decrease the amount of bending-operational force. Accordingly, an improvement in the operability during a bending operation and the durability of the wire can both be achieved by only determining the profile of the pulley, and since the component configuration does not become complex, such a configuration is extremely advantageous with regard to ease of assembly and reduction of manufacturing cost.. Furthermore, there is no risk of the control body of the endoscope becoming large or increasing in weight.

### BRIEF DESCRIPTION OF THE DRAWINGS

The present invention will be described below in detail with reference to the accompanying drawings in which:
FIG. 1 shows the entire endoscope of the illustrated embodiment;
FIG. 2 is an enlarged plan view of a bending controller, and surrounding members thereof, of the endoscope of the illustrated embodiment;
FIG. 3 shows the fundamental components of a bending control mechanism, which is provided inside the control body of the endoscope;
FIG. 4 is a cross-sectional view taken along a plane that is orthogonal to a rotational center axis of a winding section of a pulley that constitutes part of the bending control mechanism;
FIG. 5 is a cross-sectional view, similar to that of FIG. 4, showing a pulley wire that has just started to be wound onto the pulley;
FIG. 6 is a cross-sectional view, similar to that of FIG. 5, showing the pulley wire when completely wound onto the pulley;
FIG. 7 is cross-sectional view of a first comparative embodiment, which is comparative to the present invention, showing a pulley wire that has just started to be wound onto a pulley that is provided with a large-radius section over substantially the entire wire-winding area (circumference) of a winding section thereof;
FIG. 8 is a cross-sectional view showing the pulley wire when completely wound onto the pulley of the first comparative embodiment of FIG. 7;
FIG. 9 is cross-sectional view of a second comparative embodiment, which is comparative to the present invention, showing a pulley wire that has just started to be wound onto a pulley that is provided with a small-radius section over substantially the entire wire-winding area (circumference) of a winding section thereof; and
FIG. 10 is a cross-sectional view showing the pulley wire when completely wound onto the pulley of the second comparative embodiment of FIG. 9.

### DESCRIPTION OF THE PREFERRED EMBODIMENT

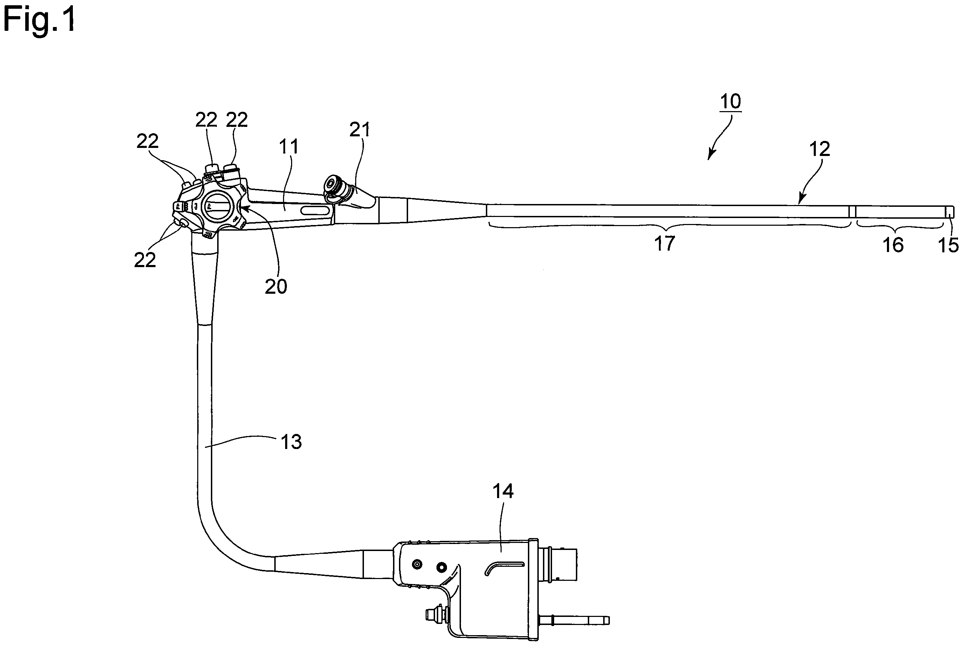

An endoscope 10 shown in FIG. 1 is provided with a control body 11, a small-diameter insertion section 12 which extends from the distal end of the control body 11, a universal cable 13 which extends from one side of the control body 11, and a connector 14 which is provided on an end of the universal cable 13. The connector 14 is connected to a processor, which is not shown in the drawings. The processor is provided with an in-built image processor and an in-built light-source lamp, for illumination purposes. The insertion section 12 is provided with a distal-end rigid section 15, a bendable section 16 which bends by being remotely controlled (operated) by the control body 11, and a flexible tube 17, in that order from the distal end of the insertion section 12. The control body 11 is provided with a bending controller 20 for controlling the bending of the bendable section 16, a treatment-tool insertion opening 21 via which a treatment tool such as a pair of forceps, etc., are inserted into a treatment-tool channel, and a plurality of control buttons 22 for sucking air from a suction inlet that is formed in the distal-end rigid section 15 and for supplying air or water, etc., from a nozzle that is formed in the distal-end rigid section 15.

The endoscope 10 is an electronic endoscope. The endoscope 10 can form an image on a light-receiving surface of an image sensor via an objective optical system, provided in the distal-end rigid section 15. Furthermore, an image signal, which is obtained from the image sensor upon being photoelectrically converted, is sent to the aforementioned image processor, provided in the processor, via a transmission cable which is provided from the insertion section 12 through to the connector 14. The image that is processed by the image processor can be displayed on a monitor display screen or can be recorded onto an image recording medium. Furthermore, a light guide is also provided from the insertion section 12 to the connector 14, and an illumination light is guided from the light source within the processor until an illumination-light lens provided within the distal-end rigid section 15 via a light guide. Furthermore, in addition to an electronic endoscope, the bending control mechanism of the illustrated embodiment can also be applied to an optical endoscope (fiberscope).

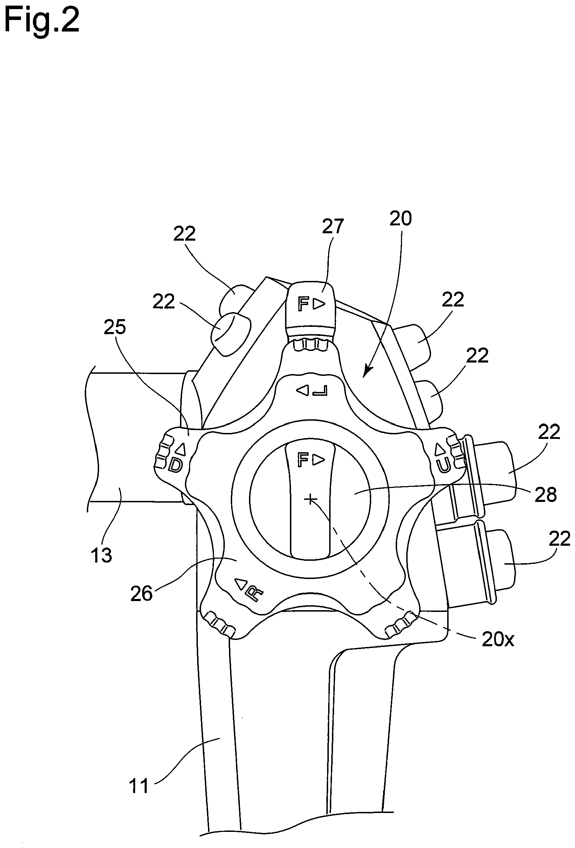

As shown in FIG. 2, the bending controller 20 is provided with an upward/downward-bending control knob 25, a leftward/rightward-bending control knob 26, a lock lever 27 and a lock knob 28. These three knobs (25, 26 and 28) and one lever (27) are each supported on the control body 11 to be independently and relatively rotatable about a common rotational center axis 20x that is shown as an imaginary axis in FIGS. 2 through 6, and overlay each other in the direction of the rotational center axis 20x. Specifically, the lock lever 27 is provided closest to an outer face of the control body 11 toward a base end of the control body 11 in the rotational center axis 20x direction, and the upward/downward-bending control knob 25 and the leftward/rightward-bending control knob 26 are placed on top of the lock lever 27 in that order, and the lock knob 28 is positioned (on top of the leftward/rightward-bending control knob 26) farthest from the outer face of the control body 11 at a forefront position in the rotational center axis 20x direction.

The bendable section 16 bends in the upward/downward direction upon the upward/downward-bending control knob 25 being manually rotated in the forward/rearward direction, and the bendable section 16 bends in the leftward/rightward direction upon the leftward/rightward-bending control knob 26 being manually rotated in the forward/rearward direction. More specifically, a downward bending operation occurs when the upward/downward-bending control knob 25 is manually rotated in a direction indicated as "D" in FIG. 2, an upward bending operation occurs when the upward/downward-bending control knob 25 is manually rotated in a direction indicated as "U" in FIG. 2, a rightward bending operation occurs when the leftward/rightward-bending control knob 26 is manually rotated in a direction indicated as "R" in FIG. 2, and a leftward bending operation occurs when the leftward/rightward-bending control knob 26 is manually rotated in a direction indicated as "L" in FIG. 2. In regard to the bending of the bendable section 16, either bending direction can be arbitrary defined as an upward/downward direction or a leftward/rightward; however, for example, bending along a plane that is horizontal to the page in FIG. 1 can be defined as the upward/downward bending direction and bending along a plane that is orthogonal to the page in FIG. 1 can be defined as the leftward/rightward bending direction. The lock lever 27 is a brake (lock) control member that restricts the rotation of the upward/downward-bending control knob 25 by the lock lever 27 being manually rotated in a direction indicated as "F" in FIG. 2. Similarly, the lock knob 28 is a brake (lock) control member that restricts the rotation of the leftward/rightward-bending control knob 26 by the lock knob 28 being manually rotated in a direction indicated as "F" in FIG. 2. The support structure for rotatably supporting the upward/downward-bending control knob 25, the leftward/rightward-bending control knob 26, the lock lever 27 and the lock knob 28 on the control body 11 in a coaxial manner is well known in the art; furthermore, the brake mechanisms for restricting the rotation of the upward/downward-bending control knob 25 and the leftward/rightward-bending control knob 26 in accordance with manual operations of the lock lever 27 and the lock knob 28, respectively, are also well known in the art; hence, descriptions of these well-known mechanisms are omitted herein.

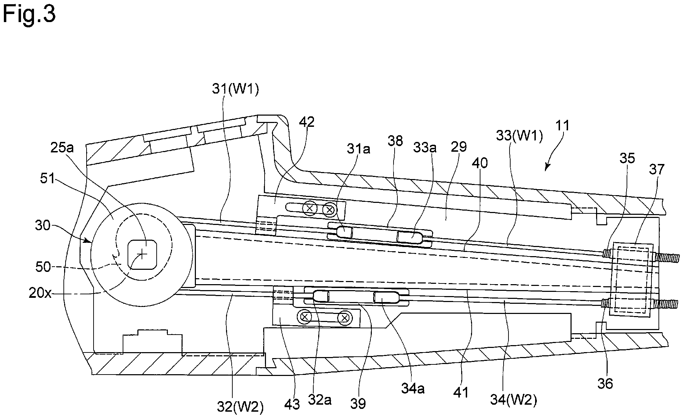

The bending control mechanism for bending the bendable section 16 via an operation of the upward/downward-bending control knob 25 (chosen out of the upward/downward-bending control knob 25 and the leftward/rightward-bending control knob 26) will be discussed hereinafter with reference to FIGS. 3 through 6.

As shown in FIG. 3, a base plate 29 is mounted inside the control body 11. The rotational center axis 20x of the bending controller 20 is an imaginary rotational center axis that extends substantially orthogonal to the plate surface of the base plate 29. A non-circular shaft 25a (e.g., a square shaft) extends from the upward/downward-bending control knob 25 along the rotational center axis 20x and into the control body 11, and a pulley 30 supported on the non-circular shaft 25a so that the pulley 30 prevented from rotating relative thereto. In other words, the pulley 30 is provided within the control body 11 and rotates integrally with the upward/downward-bending control knob 25 about the rotational center axis 20x. Common ends of a pair of pulley wires 31 and 32 are fixed to the pulley 30, and one of the pulley wires 31 and 32 is wound onto the pulley 30 and the other of the pulley wires 31 and 32 is unwound from the pulley 30 in accordance with the forward/reverse rotation of the pulley 30.

As shown in FIG. 3, a pair of angle wires 33 and 34 are also provided within the control body 11. The angle wires 33 and 34 are respectively inserted into hollow sheath coils 35 and 36 while extending inside the insertion section 12. The sections of the angle wires 33 and 34 that extend further toward the distal end than from the sheath coils 35 and 36 are not shown in FIG. 3. The sheath coils 35 and 36 are mounted onto the base plate 29 via a mounting frame 37. A plurality of joint rings (not shown) which are relatively and rotatably interconnected with each other are provided inside the bendable section 16 and are arranged in the lengthwise direction of the insertion section 12. The angle wires 33 and 34 are respectively connected to the plurality of joint rings. The distal ends of the angle wires 33 and 34 are fixed inside the distal-end rigid section 15.

As shown in FIG. 3, the pulley wire 31 and the angle wire 33 are connected to each other by a wire interconnection member 38 to constitute a continuous bending control wire W1, and the pulley wire 32 and the angle wire 34 are connected to each other by a wire interconnection member 39 to constitute a continuous bending control wire W2. Retainers 31a, 32a, 33a and 34a are provided on the ends of the pulley wires 31 and 32 and the angle wires 33 and 34, respectively. The wire interconnection member 38 and the wire interconnection member 39 are each formed as elongated box-shaped members with recesses formed therein, respectively. The retainers 31a and 33a are fitted into the recess that is formed in the wire interconnection member 38 and are held thereby, and the retainers 32a and 34a are fitted into the recess formed in the wire interconnection member 39 and are held thereby.

The wire interconnection member 38 and the wire interconnection member 39 are movable along a pair of guide surfaces 40 and 41, respectively, which are formed on the base plate 29. A stopper 42 and the sheath coil 35 are positioned in the moving path of the wire interconnection member 38 along the guide surface 40, and a stopper 43 and the sheath coil 36 are positioned in the moving path of the wire interconnection member 39 along the guide surface 41. The stoppers 42 and 43 are respectively mounted onto the base plate 29 and restrict movement of the wire interconnection members 38 and 39 in a direction toward the pulley 30. Furthermore, the surface area of each end of the wire interconnection members 38 and 39 is larger than the surface area of each opening of the sheath coils 35 and 36, respectively. Hence, the movement of the wire interconnection members 38 and 39 in a direction toward the insertion section 12 is restricted by the sheath coils 35 and 36. In other words, the mechanical movable range of the wire interconnection members 38 and 39 is respectively defined by the wire interconnection member 38 abutting against the stopper 42 and the sheath coil 35, and the wire interconnection member 39 abutting against the stopper 43 and the sheath coil 36. Furthermore, by changing the mounting positions of the stoppers 42 and 43 on the base plate 29, the maximum movement amount of the wire interconnection members 38 and 39 (while the bending control wires W1 and W2 mutually pull against each other) when the bending control wires W1 and W2 are pulled by the pulley 30, respectively, can be adjusted.

Due to the above-described configuration, upon the upward/downward-bending control knob 25 being manually rotated in the forward/reverse direction, the winding amount of the pulley wires 31 and 32 on the pulley 30 mutually increase/decrease, so that one of the bending control wires W1 and W2 is pulled and the other thereof is loosened, thereby bending the bendable section 16 in the upward/downward direction.

The pulley 30 is provided with a winding section 50, onto which the pulley wires 31 and 32 are wound onto the outer peripheral surface thereof, and a pair of flanges 51 which are positioned on either side of the winding section 50 with respect to a direction along the rotational center axis 20x (only one flange 51 is shown in each of FIGS. 3 through 6). The flanges 51 have a larger diameter than that of the winding section 50, with respect to a radial direction from the rotational center axis 20x. When the pulley wires 31 and 32 are wound onto the winding section 50, the flanges 51 prevent the pulley wires 31 and 32 from coming off (slipping off) the winding section 50 in a direction along the rotational center axis 20x.

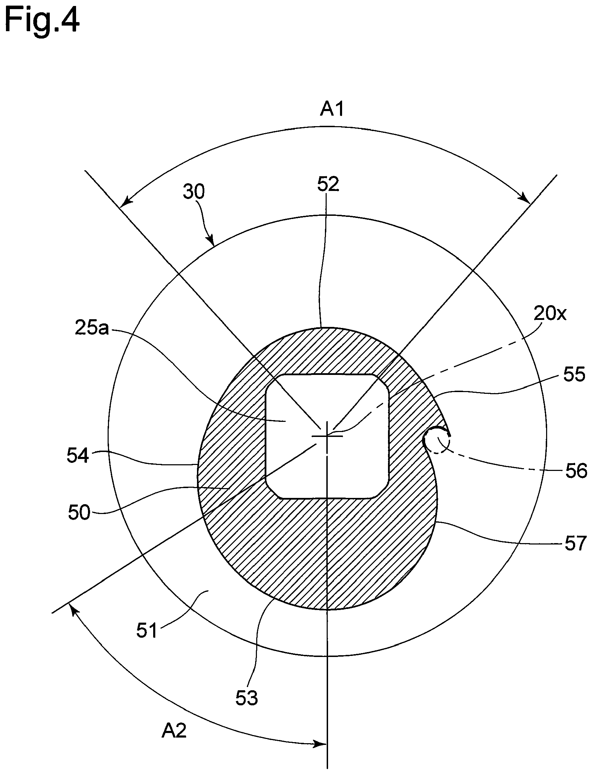

The winding section 50 of the pulley 30 is configured so that the section on which the pulley wire 31 is wound onto and the section on which the pulley wire 32 is wound onto overlap each other (are provided side by side) in a direction along the rotational center axis 20x. FIGS. 4 through 6 each show cross-section views of the section of the winding section 50 on which the pulley wire 32 is wound, however, the section on which the pulley wire 31 is wound has the same profile. More specifically, in the case where the section of the winding section 50 on which the pulley wire 32 is wound (shown in FIGS. 4 through 6) overlaps the section of the winding section 50 on which the pulley wire 31 is wound (as in the case of the illustrated embodiment), the section on which the pulley wire 31 is wound has a profile that is diametrically opposite (in a radial direction passing through the rotational center axis 20x) to the profile the section 50 on which the pulley wire 32 is wound. As can be understood from FIGS. 4 through 6, the winding section 50 has a non-circular profile (cam profile) in which the outer diameter size of the winding section 50 about the rotational center axis 20x differs at different sections thereof.

More specifically, as shown in FIG. 4, the winding section 50 has an approximately egg-shaped cross-sectional profile in a plane orthogonal to the rotational center axis 20x. The outer peripheral portion of the winding section 50 is provided with a small-radius section 52 having a small radius of curvature (the distance from the rotational center axis 20x being relatively small), a large-radius section 53 having a large radius of curvature (the distance from the rotational center axis 20x being relatively large), and a radial connecting section (varying-radius section) 54 which smoothly connects the small-radius section 52 with the large-radius section 53 without having any stepped profile therebetween. Each of the small-radius section 52 and the large-radius section 53 is a curved surface having a constant radius of curvature (constant radius section). In FIG. 4, "A1" indicates a formation range (angle) of the small-radius section 52 in a circumferential direction about the rotational center axis 20x, and "A2" indicates a formation range (angle) of the large-radius section 53 in a circumferential direction about the rotational center axis 20x. For example, it is desirable for the angle of "A1" about the rotational center axis 20x to be set to about 80 through 100 degrees, and for the angle of "A2" about the rotational center axis 20x to be set to about 50 through 70 degrees. However, the formation ranges (angles) of the small-radius section 52 and the large-radius section 53 are not limited to such angles, and can be arbitrary set to appropriate values. The radial connecting section 54 is a curved surface having a varying radius, in which the distance from the rotational center axis 20x gradually increases (i.e., the curvature gradually decreases) with respect to a circumferential direction from the small-radius section 52 to the large-radius section 53.

The winding section 50 is further provided with a gradually-varying radius section (varying-radius section) 55 and a wire lead-in section 57, in that order from the small-radius section 52, which are formed at positions located opposite to the radial connecting section 54 relative to the rotational center axis 20x. The pulley 30 is provided with a wire connector 56 (shown by an imaginary line single-dot chain line in FIGS. 4 through 6), at a circumferential position between the gradually-varying radius section 55 and the wire lead-in section 57, at a different position in a direction parallel to the rotational center axis 20x from those of the small-radius section 52, the large-radius section 53, the radial connecting section 54, the gradually-varying radius section 55 and the wire lead-in section 57. The wire connector 56 is a hole, having a substantially circular cross-section, formed along an axis extending in a direction parallel to the rotational center axis 20x. A stepped portion is formed between the gradually-varying radius section 55 and the wire lead-in section 57 at a position on an imaginary extension from the wire connector 56, in the above-mentioned direction parallel to the rotational center axis 20x. The gradually-varying radius section 55 is a curved surface having a varying radius, in which the distance from the rotational center axis 20x gradually increases (i.e., the curvature gradually decreases) with respect to a circumferential direction from the small-radius section 52 to the wire lead-in section 57. The wire lead-in section 57 is a curved surface having a varying radius, in which the distance from the rotational center axis 20x gradually increases (i.e., the curvature gradually decreases) with respect to a circumferential direction from the gradually-varying radius section 55 to the large-radius section 53.

The opposite end of the pulley wire 32 to that of the retainer 32a is inserted into the wire connector 56 and is fixedly connected thereto, and the pulley wire 32 is bent, from an insertion end of the wire connector 56, in a direction that is substantially orthogonal to the rotational center axis 20x and is guided along the wire lead-in section 57 until the large-radius section 53. In this state there is substantially no stepped section, in the radial direction of the pulley 30, between the gradually-varying radius section 55 and the pulley wire 32 that extends along the wire lead-in section 57.

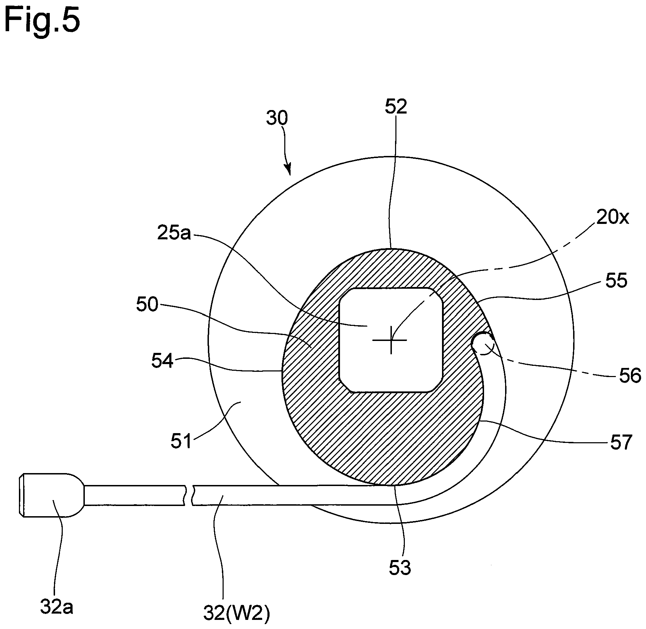

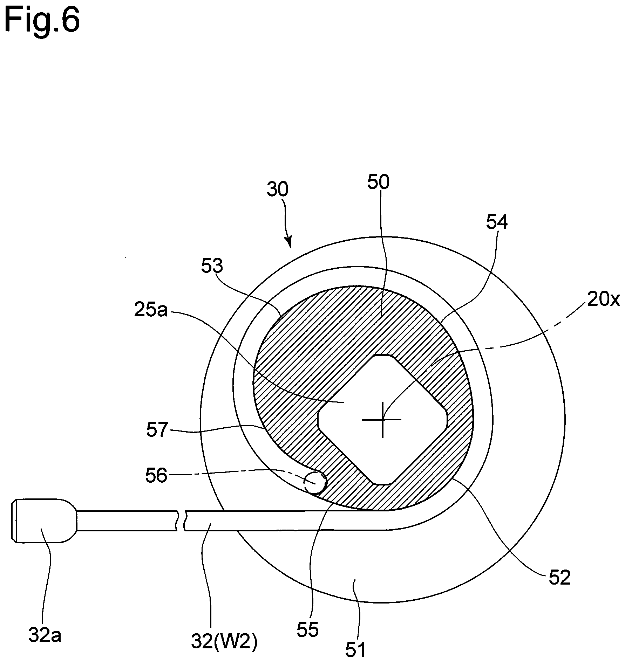

FIG. 5 shows the relationship between the winding section 50, of the pulley 30, and the pulley wire 32 in a non-bent state where the bendable section 16 is not bent in the upward or downward direction and extends in a straight line, as shown in FIG. 1. Upon the upward/downward-bending control knob 25 being manually rotated in a direction "D" (clockwise direction), shown in FIG. 2, from the not-bent state, the pulley 30 rotates in an anticlockwise direction with respect to FIG. 5. It should be noted that since FIGS. 4 through 6 view the pulley from the underside (other side) relative to the view in FIG. 2, the rotational direction of the pulley 30 in FIGS. 4 through 6 is the reverse (opposite) to the rotational direction of the upward/downward-bending control knob 25 shown in FIG. 2. Due to the rotation of the pulley 30, the end of the pulley wire 32 that is fixedly connected to the wire connector 56 is pulled while the pulley wire 32 increases its winding amount onto the winding section 50, and the retainer 32a is pulled toward the pulley 30. Consequently, the angle wire 34 which is connected to the pulley wire 32 via the wire interconnection member 39 is also pulled, so that the bendable section 16 bends downward due to the entire bending control wire W2 being pulled. When the bendable section 16 bends downward, the pulley 30 starts winding the pulley wire 32 onto the large-radius section 53 of the winding section 50 (see FIG. 5), subsequently the pulley wire 32 is wound onto and along the radial connecting section 54, and lastly the pulley wire 32 is wound onto the small-radius section 52, as shown in FIG. 6. FIG. 6 shows the relationship between the winding section 50 of the pulley 30 and the pulley wire 32 in a state where the bendable section 16 is bent to a maximum downward position. The maximum bending angle of the bendable section 16 differs depending on the endoscope, however, in the endoscope 10 of the illustrated embodiment, the bendable section 16 can be bent by approximately 180 degrees between when the pulley wire 32 starts to be wound in FIG. 5 until the winding of the pulley wire 32 has completed in FIG. 6.

Although not shown in the drawings, when the upward/downward-bending control knob 25 is manually rotated in the direction "U" (anticlockwise direction) indicated in FIG. 2 from the non-bent state (relative to the upward/downward direction) of the bendable section 16, the pulley 30 rotates in the clockwise direction, with respect to FIG. 5, and a wire winding operation similar to the relationship between the winding section 50 and the pulley wire 32 shown in FIGS. 5 and 6 is performed between the winding section 50 and the pulley wire 31. In other words, the pulley wire 31 is wound onto a large-radius section (corresponding to the large-radius section 53) of the winding section 50 at the initial stage of the bending operation, and the pulley wire 31 is wound onto a small-radius section (corresponding to the small-radius section 52) of the winding section 50 at the final stage of the bending operation.

It should be noted that although FIG. 5 shows a state of the pulley wire 32 as being wound onto the outer periphery of the winding section 50 of the pulley 30 by approximately 1/4 of the winding range (from the wire lead-in section 57 to partway along the large-radius section 53), in reality, the pulley wire 32 is wound onto the winding section 50 by another full turn to give extra allowance in a state where the bendable section 16 is not bent in the upward/downward direction, as shown in FIG. 5. As mentioned above, when the bendable section 16 is bent in the upward direction by pulling the pulley wire 31 from the state shown in FIG. 5, the pulley 30 rotates in the clockwise direction with respect to FIG. 5. Consequently, the pulley wire 32, which has been wound onto the winding section 50 by a full turn to give extra allowance, is unwound from the winding section 50 and loosened so that the pulley wire 32 does not obstruct the bending operation of the bendable section 16 in the upward direction. Similarly, the pulley wire 31, which has been wound onto the winding section 50 by a full turn to give extra allowance, is unwound from the winding section 50 and loosened so that the pulley wire 31 does not obstruct the bending operation of the bendable section 16 in the downward direction.

During the bending of the bendable section 16, as the bending angle increases, a repelling force (bending load) which tries to return the bendable section 16 to the straight position shown in FIG. 1 increases. In the bending control mechanism of the illustrated embodiment, the winding of the pulley wire 32 is carried out on the large-radius section 53 (of the winding section 50) of the pulley 30 at the initial winding stage, at which the bendable section 16 is in a non-bending position (FIG. 5). At this stage, the repelling force of the bendable section 16 against bending is small, and since only a small amount of bending operational force is required in order to rotate the upward/downward-bending control knob 25, by winding the pulley wire 32 onto the large-radius section 53 which has a large radius of curvature, the amount of stress exerted on the pulley wire 32 is reduced due to the curvature of the pulley wire 32 along the winding section 50 (large-radius section 53) being reduced, and the rotational angle of the pulley 30 (the manually rotated amount of the upward/downward-bending control knob 25) per unit of bending of the bendable section 16 can be reduced, so that the manual rotating operation does not become cumbersome. Whereas, at the stage where the bending angle of the bendable section 16 is at a maximum amount, the winding of the pulley wire 32 is carried out on the small-radius section 52 (of the winding section 50) of the pulley 30 (FIG. 6). At such a stage, although the repelling force of the bendable section 16 is at its maximum, due to the pulley wire 32 being wound onto the small-radius section 52, the amount of bending-operational force required to manually rotate the upward/downward-bending control knob 25 can be reduced, thereby reducing the operational load (the heaviness of the manual rotation of the upward/downward-bending control knob 25) for the user of the endoscope 10. Since the small-radius section 52 is only formed on part of the winding section 50 in the circumferential direction, the amount of the pulley wire 32 that is wound by the small-radius section 52 (the range in which the curvature of the pulley wire 32 along the small-radius section 52 increases) is small, thereby reducing the risk of the pulley wire 32 breaking due to stress. Furthermore, by winding the pulley wire 32 onto the radial connecting section 54 (of the winding section 50 of the pulley 30), which gradually reduces in radius, at a midway position when increasing the bending amount of the bendable section 16, which is bent from the position shown in FIG. 5 to the position shown in FIG. 6, an increase in the amount of bending-operational force can be suppressed while the pulley wire 32 can be smoothly pulled. Furthermore, since such an improvement in the operability during a bending operation and in the durability of the pulley wire 32 (31) can both be achieved by only determining the profile of the pulley 30, superior effects can be achieved, i.e., the component configuration does not become complex, assembly is facilitated and reduction of manufacturing cost can be achieved. Furthermore, since an increase in the number of components does not occur, the bending control mechanism can be provided within the same amount of space that a bending control mechanism of the related art would occupy, and hence, there is no risk of an increase in size or weight of the control body 11.

The specific configuration of the winding section 50 of the pulley 30 can be determined in view of various factors such as the thickness and material quality of the pulley wires 31 and 32, which are wound onto the pulley 30, the size and material quality of the pulley 30, and the amount of pulled required by the pulley wires 31 and 32 in order to carry out a bending operation, etc. In particular, factors such as the radius and curvature of the small-radius section 52, the radius and curvature of the large-radius section 53 play a major role in the above-mentioned operability and the durability of the pulley wire 32 (31). For example, in the case where the bending control mechanism of the present invention is applied to a typical digestive organ endoscope, it is desirable for the ratio of the radius (from the rotational center axis 20x) of the small-radius section 52 to the radius (from the rotational center axis 20x) of the large-radius section 53 to be in a range of 1:1.3 through 1:1.7, and preferably to be in a range of 1:1.4 through 1:1.6. A ratio outside such ranges runs a risk of the reduction of the amount of bending-operational force being out of balance with the reduction in the operational load for the user of the endoscope.

Although the above-descriptions have been directed to a bending control mechanism using the upward/downward-bending control knob 25 as a control member, a bending control mechanism using the leftward/rightward-bending control knob 26 as a control member is also provided with a similar configuration. Although a specific configuration is not shown in the drawings and a detailed description thereof is omitted herein, a second pulley, which is arranged beside the pulley 30 in a direction along the rotational center axis 20x, is supported in the control body 11, and a pair of pulley wires (corresponding to the pulley wires 31 and 32) are connected to the second pulley. These pulley wires are respectively connected to angle wires (corresponding to the angle wires 33 and 34) inside the control body 11, and these angle wires extend inside the insertion section 12 and are connected to a plurality of joint rings in the bendable section 16. Similar to the pulley 30, the second pulley is also provided with a winding section (corresponding to the winding section 50) having a non-circular profile including a small-radius section (corresponding to the small-radius section 52) and a large-radius section (corresponding to the large-radius section 53), and the pulley wires are similarly wound onto the winding section. The second pulley is rotated upon the leftward/rightward-bending control knob 26 being manually rotated. Accordingly, the same effects can be exhibited with a bending control mechanism using the leftward/rightward-bending control knob 26 as a control member as those with a bending control mechanism using the upward/downward-bending control knob 25 as a control member.

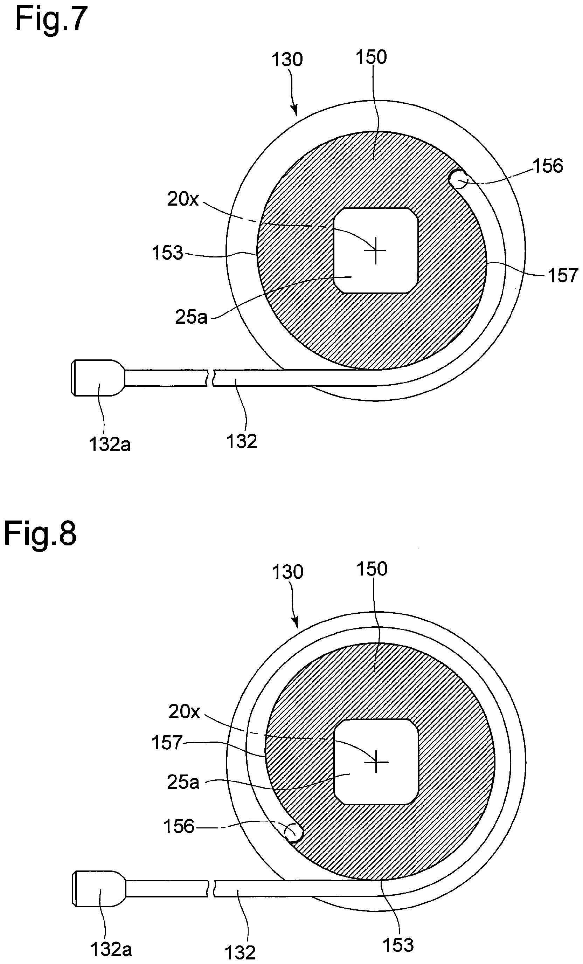

Comparative embodiments which differ from the above illustrated embodiment of the present invention are shown in FIGS. 7 through 10. In FIGS. 7 through 10, the rotational center axis 20x and the non-circular shaft 25a are the same as those of the above illustrated embodiment.

In a first comparative embodiment shown in FIGS. 7 and 8, a pulley 130 is provided with a wire connector 156, which is a hole having a substantially circular cross-section, into which an opposite end of a pulley wire 132 to that of the retainer 132a is inserted. A winding section 150, provided on the pulley 130 at a different position in the direction of the rotational center axis 20x to that of the wire connector 156, has an outer peripheral profile that includes a large-radius section 153, which has substantially the same radius as that of the large-radius section 53 of the pulley 30 of the above-described embodiment, and a wire lead-in section 157 which is formed from a position on an imaginary extension from the wire connector 156 until the large-radius section 153. The pulley wire 132, an end of which is fixedly inserted into the wire connector 156, is bent in a direction approximately orthogonal to the rotational center axis 20x and is guided along the wire lead-in section 157 until the large-radius section 153. In this pulley 130, the pulley wire 132 is always wound onto the large-radius section 153 from the initial stage of the bending operation of a bendable section (corresponding to the bendable section 16) (an initial winding stage of the pulley wire 132 onto the winding section 150, as shown in FIG. 7), until the bendable section is at a maximum bending position (a stage in which the pulley wire 132 is wound onto the winding section 150 by a maximum amount, as shown in FIG. 8). Accordingly, since the entire winding section 150 of the pulley 130 has a large radius (that is substantially the same as the large-radius section 153), when the bending angle of the bendable section becomes large so that the repelling force thereof increases, the amount of bending operational force for rotating the pulley 130 cannot be reduced and the operational load for the user of the endoscope remains large (the operational load is increased compared to the above-illustrated embodiment).

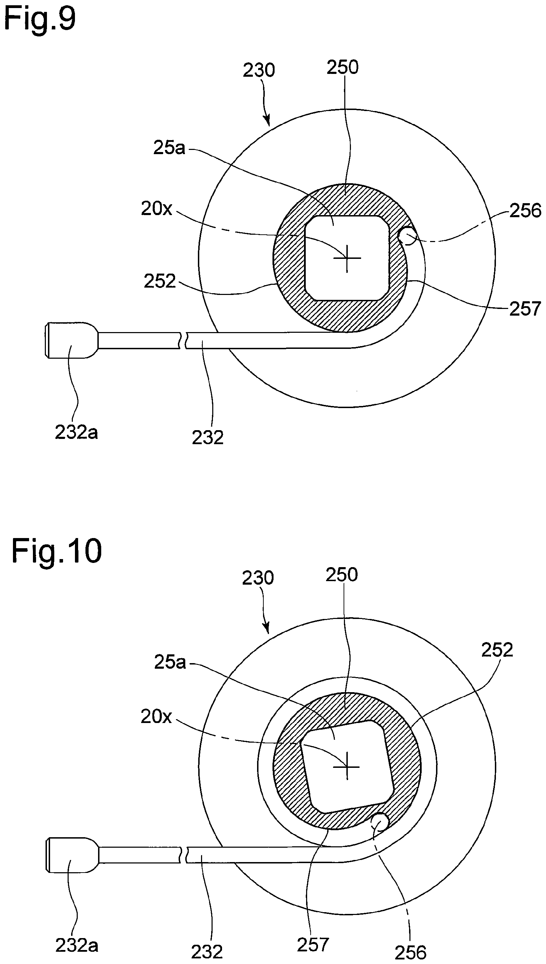

In a second comparative embodiment shown in FIGS. 9 and 10, a pulley 230 is provided with a wire connector 256, which is a hole having a substantially circular cross-section, into which an opposite end of a pulley wire 232 to that of the retainer 232a is inserted. A winding section 250, provided on the pulley 230 at a different position in the direction of the rotational center axis 20x to that of the wire connector 256, has an outer peripheral profile that includes a small-radius section 252, which has substantially the same radius as that of the small-radius section 52 of the pulley 30 of the above-described embodiment, and a wire lead-in section 257 which is formed from a position on an imaginary extension from the wire connector 256 until the small-radius section 252. The pulley wire 232, an end of which is fixedly inserted into the wire connector 256, is bent in a direction approximately orthogonal to the rotational center axis 20x and is guided along the wire lead-in section 257 until the small-radius section 252. In this pulley 230, the pulley wire 232 is always wound onto the small-radius section 252 from the initial stage of the bending operation of a bendable section (corresponding to the bendable section 16) (an initial winding stage of the pulley wire 232 onto the winding section 250, as shown in FIG. 9), until the bendable section is at a maximum bending position (a stage in which the pulley wire 232 is wound onto the winding section 250 by a maximum amount, as shown in FIG. 10). It should be noted that although FIG. 10 shows part of the pulley wire 232 overlapping itself in the radial direction, since the radially inner pulley wire 232 positioned at this overlapped section is positioned along the wire lead-in section 257, the radially outer pulley wire 232 positioned along this overlapped section has substantially the same curvature as when the pulley wire 232 is wound onto the small-radius section 252. Accordingly, since the entire winding section 250 of the pulley 230 has a small radius (that is substantially the same as a small-radius section 252), due to the pulley wire 232 being repeatedly extended and bent at a small radius of curvature, fatigue occurs, thereby increasing the risk of the pulley wire 232 breaking. Furthermore, since the rotational angle of the pulley 230 that is required to bend the bendable section by a predetermined amount of bending is always large, thereby causing a cumbersome manual operation (of the upward/downward-bending control knob or leftward/rightward-bending control knob).

Although the present invention has be described based on the above illustrated embodiment, the present invention is not limited thereto; various modifications can be made that are within the spirit and scope of the present invention. For example, the illustrated embodiment is provided with two pulleys (the pulley 30 and the second pulley) which are manually rotated via the upward/downward-bending control knob 25 and the leftward/rightward-bending control knob 26, respectively; however, the present invention can be applied using a different number of pulleys that are rotated during a bending operation. Namely, it is possible to apply the bending control mechanism of the endoscope 10 using one or three or more pulleys. Furthermore, in the illustrated embodiment, although a pair of pulley wires 31 and 32 are connected to the pulley 30, the present invention can be applied to a type of bending control mechanism which only has one pulley wire connected to a pulley (e.g., with only the pulley wire 32 shown in FIGS. 5 and 6 being connected to the pulley 30 and a pulley wire corresponding to the pulley wire 31 omitted) . Furthermore, it is also possible to use a lever, etc., as a control member for manually rotating the pulley instead of the upward/downward-bending control knob 25 and/or the leftward/rightward-bending control knob 26.

The pulley 30 of the illustrated embodiment is provided with the winding section 50 having a profile including the small-radius section 52 and the large-radius section 53, each having a constant curvature (a radius of curvature from the rotational center axis 20x), and the radial connecting section 54 connecting the small-radius section 52 with the large-radius section 53.

## Claims

1. A bending control mechanism for an endoscope (10), provided with a control body (11) and a bendable section (16) which includes an insertion section (12), said bending control mechanism comprising:
a pulley (30) which is rotatably provided in said control body (11); and
a wire (W1, W2) which is connected between said pulley (30) and said bendable section (16),
wherein an amount of said wire (W1, W2) is wound onto said pulley (30) in accordance with a change in rotation of said pulley (30),
**characterized in that** an outer peripheral profile of said pulley (30), onto which said wire (W1, W2) is wound, includes a non-circular section (50) including a small-radius section (52) having a small radius from a rotational center axis (20x) of said pulley (30), and a large-radius section (53) having a radius from said rotational center axis (20x) that is larger than said small radius,
wherein said wire (W1, W2) is wound onto said large-radius section (53) at an initial winding stage at which said bendable section (16) is at a non-bending position,
wherein said wire (W1, W2) is wound onto said small-radius section (52) at a stage where said bendable section (16) is bent by a maximum amount, and
wherein each of said small-radius section (52) and said large-radius section (53) of said pulley (30) comprises a constant radius section having a constant radius from said rotational center axis (20x).

2. The bending control mechanism for an endoscope according to claim 1, wherein said pulley (30) comprises a varying-radius section (54) between said small-radius section (52) and said large-radius section (53), wherein a radius of said varying-radius section (54) from said rotational center axis (20x) gradually increases with respect to a direction from said small-radius section (52) to said large-radius section (53) .

3. The bending control mechanism for an endoscope according to claim 2, wherein said varying-radius section (54) comprises a radial connecting section which smoothly connects said small-radius section (52) with said large-radius section (53) without having any stepped profile therebetween, and
wherein an end of said wire (W1, W2) is fixed to said pulley (30) at a position within a range, in a circumferential direction of said pulley (30), other than said small-radius section (52), said large-radius section (53) and said radial connecting section.

## Patentansprüche

1. Biegesteuerungsmechanismus für ein Endoskop (10), versehen mit einem Steuerkörper (11) und einem Biegeabschnitt (16), der einen Einführabschnitt (12) aufweist, wobei der Biegesteuerungsmechanismus umfasst:
eine Rolle (30), die drehbar in dem Steuerkörper (11) vorgesehen ist; und
einen Draht (W1, W2), der zwischen der Rolle (30) und dem Biegeabschnitt (16) vorgesehen ist,
wobei ein Anteil des Drahtes (W1, W2) entsprechend einer Änderung der Drehung der Rolle (30) auf die Rolle (30) gewickelt wird,
**dadurch gekennzeichnet, dass** ein Außenumfangsprofil der Rolle (30), auf das der Draht (W1, W2) gewickelt ist, einen nicht-kreisförmigen Abschnitt (50) mit einem radiuskleinen Abschnitt (20), der ausgehend von einer Drehmittelachse (20x) der Rolle (30) einen kleinen Radius hat, und einem radiusgroßen Abschnitt (53), der ausgehend von der Drehmittelachse (20x) einen Radius hat, der größer als der kleine Radius ist,
wobei der Draht (W1, W2) in einem initialen Wickelstadium, in dem der Biegeabschnitt (16) in einer nicht-gebogenen Stellung ist, auf den radiusgroßen Abschnitt (53) gewickelt wird,
wobei der Draht (W1, W2) in einem Stadium, in dem der Biegeabschnitt um einen maximalen Betrag gebogen ist, auf den radiuskleinen Abschnitt (52) gewickelt wird, und
wobei der radiuskleine Abschnitt (52) und der radiusgroße Abschnitt (53) der Rolle (30) jeweils einen radiuskonstanten Abschnitt aufweisen, der ausgehend von der Drehmittelachse (20x) einen konstanten Radius hat.

2. Biegesteuerungsmechanismus für ein Endoskop nach Anspruch 1, wobei die Rolle (30) einen radiusvariierenden Abschnitt (54) zwischen dem radiuskleinen Abschnitt (52) und dem radiusgroßen Abschnitt (53) hat, wobei ein Radius des radiusvariierenden Abschnittes (54) ausgehend von der Drehmittelachse (20x) bezogen auf eine Richtung ausgehend von dem radiuskleinen Abschnitt (52) zu dem radiusgroßen Abschnitt (53) allmählich zunimmt.

3. Biegesteuerungsmechanismus für ein Endoskop nach Anspruch 2, wobei der radiusvariierende Abschnitt (54) einen radial verbindenden Abschnitt aufweist, der den radiuskleinen Abschnitt (52) glatt mit dem radiusgroßen Abschnitt (53) verbindet, ohne ein gestuftes Profil dazwischen aufzuweisen, und
ein Ende des Drahtes (W1, W2) an der Rolle (30) an einer Stelle innerhalb eines Bereichs in Umfangsrichtung der Rolle (30) befestigt ist, der nicht den radiuskleinen Abschnitt (52), den radiusgroßen Abschnitt (53) und den radial verbindenden Abschnitt beinhaltet.

## Revendications

1. Mécanisme de commande flexible pour un endoscope (10), muni d'un corps de commande (11) et d'un segment cintrable (16) qui inclut un segment d'insertion (12), ledit mécanisme de commande flexible comprenant :
une poulie (30) qui est montée pour tourner dans ledit corps de commande (11) ; et
un fil (W1, W2) qui est raccordé entre ladite poulie (30) et ledit segment cintrable (16),
dans lequel une certaine longueur dudit fil (W1, W2) est enroulée sur ladite poulie (30) en fonction d'un changement dans la rotation de ladite poulie (30),
**caractérisé en ce qu'**un profil périphérique extérieur de ladite poulie (30), sur laquelle ledit fil (W1, W2) est enroulé, inclut un segment non circulaire (50), comprenant un segment à petit rayon (52) ayant un petit rayon à partir d'un axe central de rotation (20x) de ladite poulie (30), et un segment à grand rayon (53) ayant un rayon à partir dudit axe central de rotation (20x) qui est plus grand que ledit petit rayon,
dans lequel ledit fil (W1, W2) est enroulé sur ledit segment à grand rayon (53) à un niveau d'enroulement initial auquel ledit segment cintrable (16) est dans une position non flexible,
dans lequel ledit fil (W1, W2) est enroulé sur ledit segment à petit rayon (52) à un niveau où ledit segment cintrable (16) est cintré suivant une amplitude maximum, et
dans lequel chacun dudit segment à petit rayon (52) et dudit segment à grand rayon (53) de ladite poulie (30) comprend un segment à rayon constant ayant un rayon constant à partir dudit axe central de rotation (20x).

2. Mécanisme de contrôle flexible pour un endoscope selon la revendication 1, dans lequel ladite poulie (30) comprend un segment à rayon variable (54) entre ledit segment à petit rayon (52) et ledit segment à grand rayon (53), dans lequel un rayon dudit segment à rayon variable (54) à partir dudit axe central de rotation (20x) augmente progressivement par rapport à une direction allant dudit segment à petit rayon (52) au dit segment à grand rayon (53).

3. Mécanisme de commande flexible pour un endoscope selon la revendication 2, dans lequel ledit segment à rayon variable (54) comprend un segment de raccordement radial qui raccorde facilement ledit segment à petit rayon (52) avec ledit segment à grand rayon (53) sans qu'il y ait aucun profil étagé entre ceux-ci, et
dans lequel une extrémité dudit fil (W1, W2) est fixée à ladite poulie (30) dans une position, dans une certaine plage, suivant la direction de la circonférence de ladite poulie (30), autre que ledit segment à petit rayon (52), ledit segment à grand rayon (53) et ledit segment de raccordement radial.
